# EUROPEAN PATENT APPLICATION

(11) **EP 1 336 399 A2**
(43) Date of publication of application: **20.08.2003**
(21) Application number: 03250926.7
(22) Date of filing: 14.02.2003
(51) Int. Cl.: A61F 13/537

(54) **Three-dimensional apertured film**

(30) Priority: 14.02.2002 US 356837 P
(71) Applicant: McNeil-PPC, Inc., Skillman, NJ 08558-9418 (US)
(72) Inventor: Kelly, William G.F., Middlesex, NJ 08846 (US); James, William A., Pennington 08534 (US)
(74) Representative: Mercer, Christopher Paul

(57) **Abstract**

This invention provides a three-dimensional apertured film having a caliper defined by a first plane and a second plane. The film comprises a plurality of disconnected macrofeatures and a plurality of apertures. The apertures are defined by sidewalls that originate in the film's first surface and extend generally in the direction of the film's second surface to terminate in the second plane. The first surface of the film is coincident with the first plane at said macrofeatures. The film may be used in personal care products, such as sanitary napkins, and has stable, low Penetration Rate when used in conjunction with an absorbent core containing superabsorbent material.

## Description

### Field of the Invention

The present invention relates generally to three-dimensional apertured film materials useful as components of personal care products such as sanitary napkins, diapers, incontinence products, tampons, surgical dressings, wound dressings, underpads, wiping cloths, and the like. In particular, the invention relates to three-dimensional apertured polymeric films with improved fluid-handling properties when used as a component layer in disposable absorbent products.

### Summary of the Invention

The present invention provides a three-dimensional apertured film with a first surface, a second surface, and a caliper defined by a first plane and a second plane. The film comprises a plurality of disconnected macrofeatures and a plurality of apertures. The apertures are defined by sidewalls that originate in the film's first surface and extend generally in the direction of the film's second surface to terminate in the second plane. The first surface of the film is coincident with the first plane at the disconnected macrofeatures.

The relative positions of the apertures and macrofeatures in the film is regular. This means the apertures and macrofeatures are arranged in an orderly and consistent configuration relative to one another; i.e., the apertures and macrofeatures recur at fixed or uniform intervals with respect to one another. The spatial relationship between the apertures and the macrofeatures define a geometric pattern that is consistently repeated throughout the surface area of the film. The apertures and macrofeatures are arranged in a regular, defined pattern uniformly repeated throughout the film.

The film advantageously provides improved fluid management properties, as demonstrated by more consistent, and reduced fluid absorption times upon repeat insult testing. When subjected to repeated insults by means of the Repeat Insult Test, that is the repeated introduction of synthetic menstrual fluid to the same area, an absorbent article comprising a nonwoven cover layer, an intermediate layer and an absorbent core comprising superabsorbent polymer has a Repeat Insult Time which increases less than 40% over six insults. Specifically, the film of the present invention advantageously provides a Repeat Insult Time that increases less than about 40% over six insults when tested as an intermediate layer under a nonwoven cover layer and over an absorbent core containing superabsorbent polymer. As used herein,an intermediate layer is a layer of an absorbent article located immediately adjacent the cover layer and between the cover layer and absorbent core. The intermediate layer is also known as a transfer layer. In a preferred embodiment, the Repeat Insult Time increases less than about 30%; in a more preferred embodiment, the Repeat Insult Time increases less than about 20%; and, in a most preferred embodiment, the Repeat Insult Time increases less than about 15% over six insults.

### Brief Description of the Drawings

Figure 1 is a photomicrograph of an embodiment of a three-dimensional film of the present invention.
Figure 1A is an illustration of a cross-section of the film of Figure 1 along line A-A.
Figure 2 is a photomicrograph of another embodiment of a three-dimensional film of the present invention.
Figure 2A is an illustration of a cross-section of the film of Figure 2 along line A-A.
Figure 2B is an illustration of a cross-section of the film of Figure 2 along line B-B.
Figure 3 is a photomicrograph of yet another embodiment of a three-dimensional film of the present invention. Figure 3A is an illustration of a cross-section of the film of Figure 3 along line A-A.
Figure 4 is a photomicrograph of another embodiment of a three-dimensional film of the present invention.
Fig. 5 is a schematic illustration of one type of three dimensional topographical support member useful to make a film of the present invention.
Fig. 6 is a schematic illustration of an apparatus for laser sculpting a workpiece to form a three dimensional topographical support member useful to make a film of the present invention.
Fig. 7 is a schematic illustration of a computer control system for the apparatus of Figure 6.
Fig. 8 is a graphical enlargement of an example of a pattern file to raster drill a workpiece to produce a support member for apertured film.
Fig. 9 is a photomicrograph of a workpiece after it has been laser drilled using the file of Fig. 8.
Fig. 10 is a graphical representation of a file to laser sculpt a workpiece to produce the film of Figure 2.
Fig. 11 is a graphical representation of a file to laser sculpt a workpiece to produce a three dimensional topographical support member useful to make a film of this invention.
Fig. 12 is a photomicrograph of a workpiece that was laser sculpted utilizing the file of Fig. 11.
Fig. 12A is a photomicrograph of a cross section of the laser sculpted workpiece of Fig. 12.
Fig. 13 is a photomicrograph of an apertured film produced using the laser sculpted support member of Fig. 12.
Fig. 13A is another photomicrograph of an apertured film produced using the laser sculpted support member of Fig. 12.
Fig. 14 is an example of a file which may be used to produce a support member by laser modulation.
Fig. 14A is a graphical representation of a series of repeats of the file of Fig. 14.
Fig. 15 is an enlarged view of portion B of the file of Fig. 14.
Fig. 16 is a graphical enlargement of a pattern file used to create portion C of Fig. 15.
Fig. 17 is a photomicrograph of a support member produced by laser modulation using the file of Fig. 14.
Fig. 18 is a photomicrograph of a portion of the support member of Fig. 17.
Fig. 19 is a photomicrograph of a film produced by utilizing the support member of Fig. 17.
Fig. 20 is a photomicrograph of a portion of the film of Fig. 19.
Fig. 21 is a view of a support member used to make a film according to the invention in place on a film-forming apparatus.
Fig. 22 is a schematic view of an apparatus for producing an apertured film according to the present invention.
Fig. 23 is a schematic view of the circled portion of Fig. 22.
Fig. 24 is a photomicrograph of an apertured film of the prior art.
Fig. 25 is a photomicrograph of another example of an apertured film of the prior art.
Fig. 26 is a photomicrograph of another example of an apertured film of the present invention.

### Detailed Description of the Invention

The present invention is directed to three-dimensional apertured films particularly useful in personal care products. These films may be used as body-contacting facing layers, as fluid handling layers, or as other components of personal care products. The films of the invention have been found to exhibit improved fluid-handling properties when used in disposable absorbent articles such as, for instance, feminine sanitary protection products.

The three-dimensional apertured film of the invention has a first surface and a second surface. The film additionally has a caliper defined by a first plane and a second plane. The film has a plurality of apertures defined by sidewalls that originate in the first surface and extend generally in the direction of the second surface to terminate in the second plane. The film also comprises a plurality of disconnected macrofeatures. The first surface of the film coincides with the first plane at these macrofeatures.

As used herein, the term "macrofeature" means a projection in the film visible to the normal, unaided human eye at a perpendicular distance of 12 inches between the eye and the film. The macrofeatures are arranged in a regular configuration relative to the apertures. In some embodiments, for example, the apertures may alternate with the macrofeatures. In other embodiments, the apertures and macrofeatures may be arranged so chat there are more apertures than macrofeatures, although the relative arrangement of apertures and macrofeatures is regular. The exact sizes and shapes of the apertures and macrofeatures are not critical, as long as the macrofeatures are large enough to be visible to a normal unaided human eye at a distance of 12 inches, and as long as the macrofeatures are discrete and disconnected from one another.

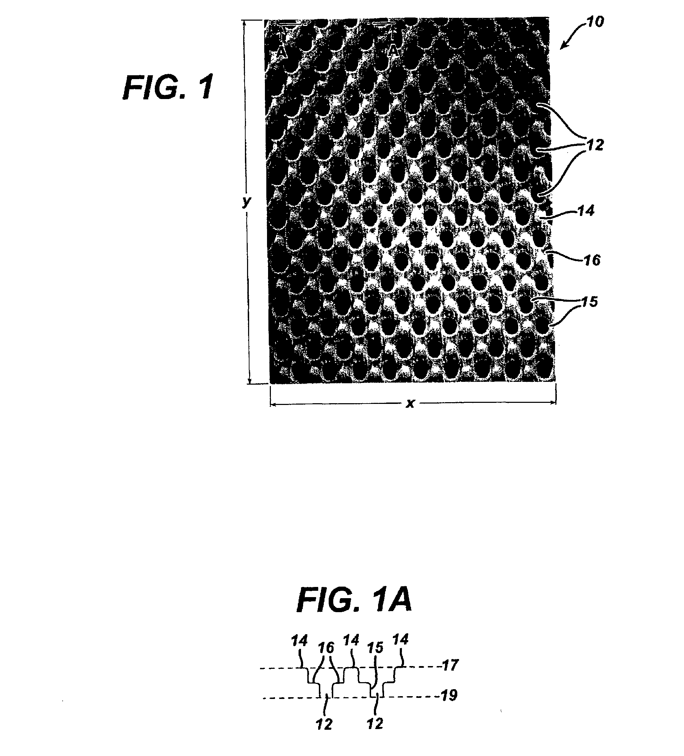

Figure 1 is a photomicrograph of an embodiment of a three-dimensional apertured film according to the present invention. The film 10 of Figure 1 has apertures 12 and macrofeatures 14. The apertures are defined by sidewalls 15. The macrofeatures are discrete projections in the film and can be seen to project above lower regions 16 of the first surface. If an imaginary plane, i.e., a first plane, were lowered onto the first surface of the three-dimensional apertured film, it would touch the film at the top of the macrofeatures in multiple discrete areas separated from one another. It is not necessary for each and every macrofeature to touch the imaginary plane; rather, the first plane is thus defined by the uppermost portions of the macrofeatures, that is, those parts of the macrofeatures projecting the farthest from the second surface of the film.

In the embodiment of Figure 1, the apertures alternate with the macrofeatures in both the x-direction and the y-direction, and the ratio of apertures to macrofeatures is one.

Figure 1A is an illustration of a cross-section of the film 10 of Figure 1 along line A-A of Figure 1. As Figure 1A shows, the macrofeatures 14 are disconnected from one another in first plane 17 and are separated from one another by lower regions 16 of the first surface of the film and by apertures 12. The apertures 12 are defined by sidewalls 15 which originate in the first surface and extend generally in the direction of the second surface to terminate in second plane 19. It is not necessary for all of the apertures to terminate in the second plane 19; rather, the second plane is defined by the lowermost extending sidewalls 15.

In one embodiment of the invention, at least a portion of the apertures have sidewalls having a first portion that originates in the first plane of the film and a second portion that originates in a plane located between the first and second planes of the film, that is a plane intermediate the first and second planes.

In a preferred embodiment, in addition to having apertures with sidewalls having first portions originating in the first plane and second portions originating in an intermediate plane, the film comprises apertures whose sidewalls originate completely in an intermediate plane. That is, the film contains apertures that originate in a plane other than the plane defined by the uppermost surface of the macrofeatures.

In a particularly preferred embodiment of the present invention, the film comprises a combination of several different types of apertures. The film comprises apertures whose sidewalls originate in the first plane of the film. The film also comprises apertures having sidewalls, a portion of which originate in the first plane and a portion of which originate in an intermediate plane. Finally, the film also comprises apertures whose sidewalls originate completely in an intermediate plane.

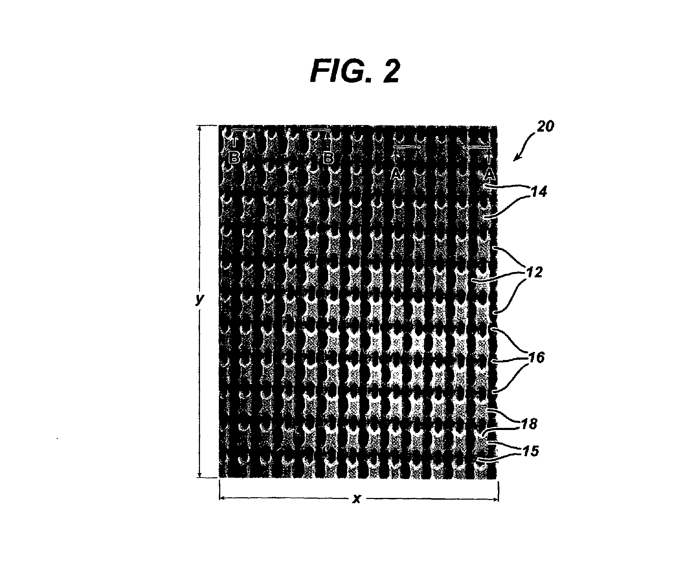

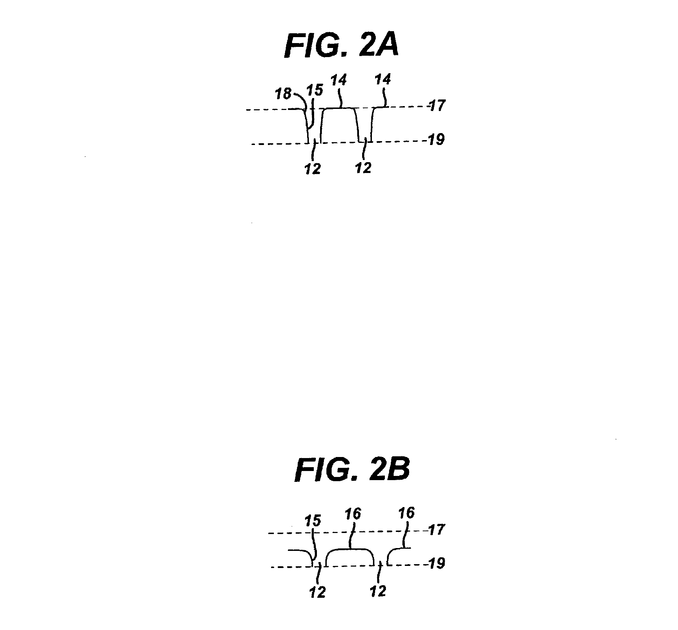

In Figure 2, apertures 12 are defined by sidewalls 15. The macrofeatures 14 project above lower regions 16 of the first surface of the film 20. The macrofeatures and apertures are shaped differently from the macrofeatures and apertures of the film of Figure 1. In Figure 2, the macrofeatures are separated from one another by apertures in the x-direction and in the y-direction. However, some of the apertures are separated from one another by lower regions 16 of the first surface in both the x-direction and the y-direction. In the film 20 of Figure 2, the ratio of apertures to macrofeatures is 2.0. Moreover, each aperture in the film 20 of Figure 2 has a portion of its sidewall originating in the first plane 17, shown in Figure 2A, i.e., at an edge 18 of a macrofeature, and a portion of its sidewall originating in a lower region 16 of the first surface.

Figure 2A shows a cross-section of the film 20 of Figure 2 along line A-A. The macrofeatures 14 are separated from one another in the first plane 17 by apertures 12, which are defined by sidewalls 15 that originate in the first surface of the film and extend generally in the direction of the second surface to terminate in the second plane 19. It can be seen in Figure 2A that the portions of the sidewalls 15 shown in this cross-section originate in the first plane 17 at the edges 18 of the macrofeatures 14.

Figure 2B shows a cross-section of the film 20 of Figure 2 taken along line B-B. In this particular cross-section, no macrofeatures are visible, and the apertures 12 are separated from one another by lower regions 16 of the first surface of the film. The lower regions 16 of the film lie between the first plane 17 and the second plane 19, said planes defining the caliper of the three-dimensional apertured film shown. The sidewalls 15 terminate in the second plane 19.

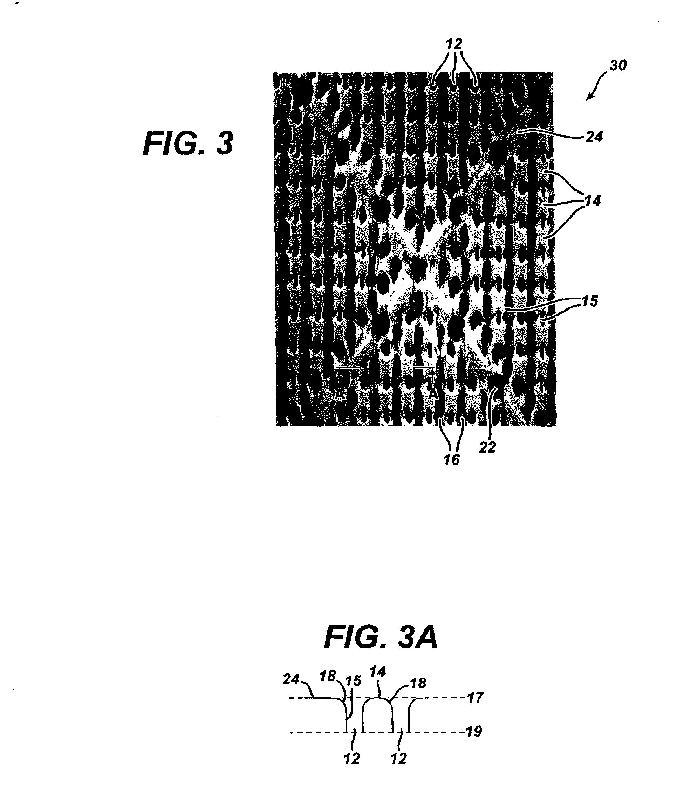

Figure 3 shows a photomicrograph of a further embodiment of a three-dimensional apertured film of the present invention with yet another arrangement of apertures and macrofeatures. The film 30 of Figure 3 has apertures 12 arranged with macrofeatures 14, and apertures 22 arranged with macrofeatures 24. All of the apertures 12, 22 and macrofeatures 14, 24 are arranged together so that their relative positions to one another are regular.

Figure 3A is a cross-section of the film 30 of Figure 3 taken along line A-A of Figure 3. This particular cross-section shows macrofeatures 24 and macrofeatures 14 disconnected from one another in first plane 17 and separated from one another by apertures 12. The apertures 12 are defined by sidewalls 15 that terminate in the second plane 19. The portions of the sidewalls 15 shown in this particular cross-section originate in the first plane 17 at the edges 18 of the macrofeatures 14 and 24.

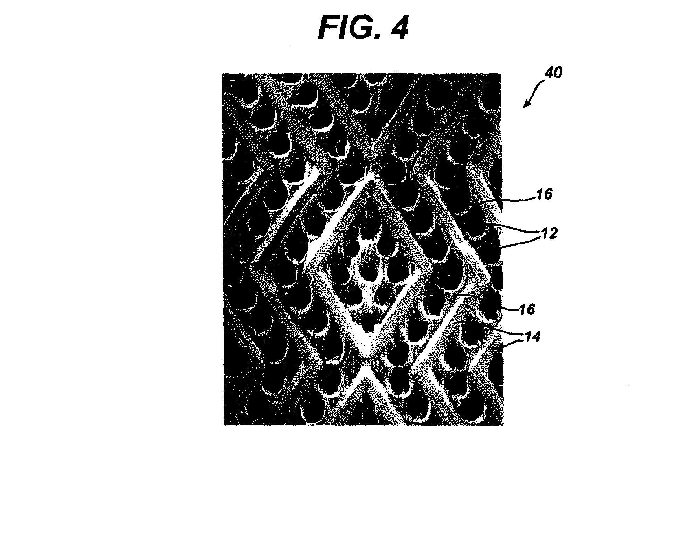

Figure 4 is a photomicrograph of yet another embodiment of a three-dimensional apertured film according to the present invention. The film 40 shown in Figure 4 has a regular arrangement of apertures 12 and macrofeatures 14.

A suitable starting film is a thin, continuous, uninterrupted film of thermoplastic polymeric material. This film may be vapor permeable or vapor impermeable; it may be embossed or unembossed; it may be corona-discharge treated on one or both of its major surfaces or it may be free of such corona-discharge treatment; it may be treated with a surface active agent after the film is formed by coating, spraying, or printing the surface active agent onto the film, or the surface active agent may be incorporated as a blend into the thermoplastic polymeric material before the film is formed. The film may comprise any thermoplastic polymeric material including, but not limited to, polyolefins, such as high density polyethylene, linear low density polyethylene, low density polyethylene, polypropylene; copolymers of olefins and vinyl monomers, such as copolymers of ethylene and vinyl acetate or vinyl chloride; polyamides; polyesters; polyvinyl alcohol and copolymers of olefins and acrylate monomers such as copolymers of ethylene and ethyl acrylate and ethylenemethacrylate. Films comprising mixtures of two or more of such polymeric materials may also be used. The machine direction (MD) and cross direction (CD) elongation of the starting film to be apertured should be at least 100% as determined according to ASTM Test No. D-882 as performed on an Instron test apparatus with a jaw speed of 50 inches/minute (127 cm/minute). The thickness of the starting film is preferably uniform and may range from about 0.5 to about 5 mils or about 0.0005 inch (0.0013 cm) to about 0.005 inch (0.076 cm). Coextruded films can be used, as can films that have been modified, e.g., by treatment with a surface active agent. The starting film can be made by any known technique, such as casting, extrusion, or blowing.

A method of aperturing the film of the invention involves placing the film onto the surface of a patterned support member. The film is subjected to a high fluid pressure differential as it is on the support member. The pressure differential of the fluid, which may be liquid or gaseous, causes the film to assume the surface pattern of the patterned support member. If the patterned support member has apertures therein, portions of the film overlying the apertures may be ruptured by the fluid pressure differential to create an apertured film. A method of forming an apertured film is described in detail in commonly owned US 5,827,597 to James et al., incorporated herein by reference.

An apertured film of this invention is preferably formed by placing a thermoplastic film across the surface of an apertured support member with a pattern of macrofeatures and apertures. A stream of hot air is directed against the film to raise its temperature to cause it to be softened. A vacuum is then applied to the film to cause it to conform to the shape of the surface of the support member. Portions of the film lying over the apertures in the support member are ruptured to create apertures in the film.

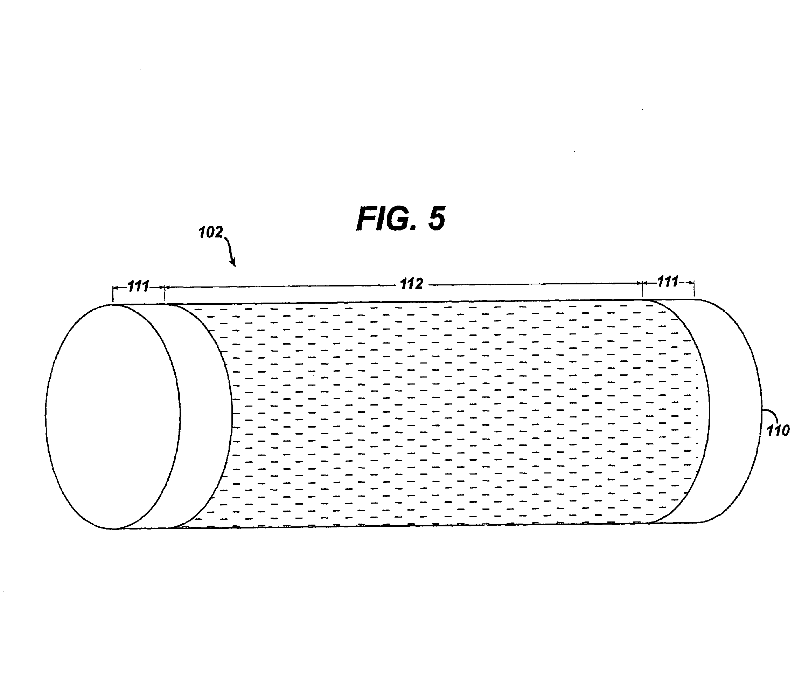

A suitable apertured support member for making the three-dimensional apertured films of the present invention is a three-dimensional topographical support member made by laser sculpting a workpiece. A schematic illustration of an exemplary workpiece that has been laser sculpted into a three dimensional topographical support member is shown in Figure 5.

The workpiece 102 comprises a thin tubular cylinder 110. The workpiece 102 has non-processed surface areas 111 and a laser sculpted center portion 112. A preferred workpiece for producing the support member of this invention is a thin-walled seamless tube of acetal, which has been relieved of all residual internal stresses. The workpiece has a wall thickness of from 1-8 mm, more preferably from 2.5-6.5 mm. Exemplary workpieces for use in forming support members are one to six feet in diameter and have a length ranging from two to sixteen feet. However, these sizes are a matter of design choice. Other shapes and material compositions may be used for the workpiece, such as acrylics, urethanes, polyesters, high molecular weight polyethylene and other polymers that can be processed by a laser beam.

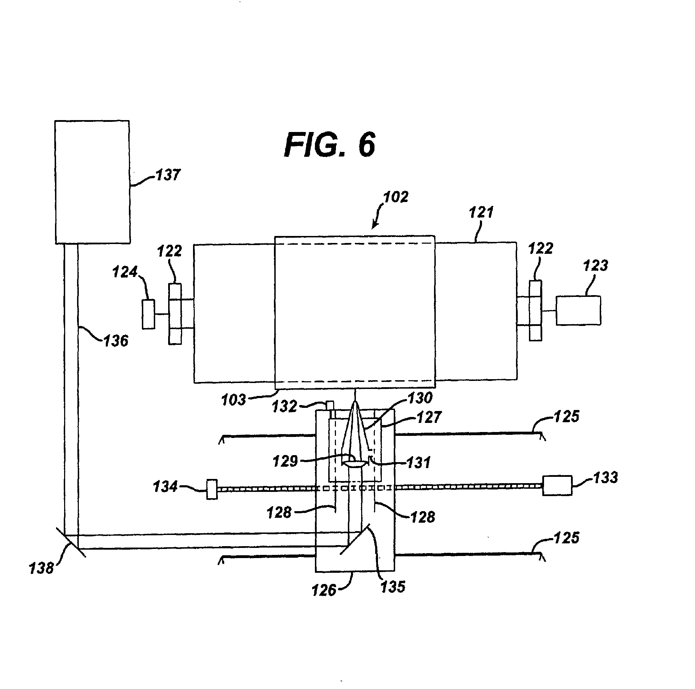

Referring now to Fig. 6, a schematic illustration of an apparatus for laser sculpting the support member of this invention is shown. A starting blank tubular workpiece 102 is mounted on an appropriate arbor, or mandrel 121 that fixes it in a cylindrical shape and allows rotation about its longitudinal axis in bearings 122. A rotational drive 123 is provided to rotate mandrel 121 at a controlled rate. Rotational pulse generator 124 is connected to and monitors rotation of mandrel 121 so that its precise radial position is known at all times.

Parallel to and mounted outside the swing of mandrel 121 is one or more guide ways 125 that allow carriage 126 to traverse the entire length of mandrel 121 while maintaining a constant clearance to the top surface 103 of workpiece 102. Carriage drive 133 moves the carriage along guide ways 125, while carriage pulse generator 134 notes the lateral position of the carriage with respect to workpiece 102. Mounted on the carriage is focusing stage 127. Focusing stage 127 is mounted in focus guide ways 128. Focusing stage 127 allows motion orthogonal to that of carriage 126 and provides a means of focusing lens 129 relative to top surface 103. Focus drive 132 is provided to position the focusing stage 127 and provide the focusing of lens 129.

Secured to focusing stage 127 is the lens 129, which is secured in nozzle 130. Nozzle 130 has means 131 for introducing a pressurized gas into nozzle 130 for cooling and maintaining cleanliness of lens 129. A preferred nozzle 130 for this purpose is described in US Patent 5,756,962 to James et al. which is incorporated herein by reference.

Also mounted on the carriage 126 is final bending mirror 135, which directs the laser beam 136 to the focusing lens 129. Remotely located is the laser 137, with optional beam bending mirror 138 to direct the beam to final beam bending mirror 135. While it would be possible to mount the laser 137 directly on carriage 126 and eliminate the beam bending mirrors, space limitations and utility connections to the laser make remote mounting far preferable.

When the laser 137 is powered, the beam 136 emitted is reflected by first beam bending mirror 138, then by final beam bending mirror 135, which directs it to lens 129. The path of laser beam 136 is configured such that, if lens 129 were removed, the beam would pass through the longitudinal center line of mandrel 121. With lens 129 in position, the beam may be focused above, below, at, or near top surface 103.

While this invention could be used with a variety of lasers, the preferred laser is a fast flow CO₂ laser, capable of producing a beam rated at up to 2500 watts. However, slow flow CO₂ lasers rated at 50 watts could also be used.

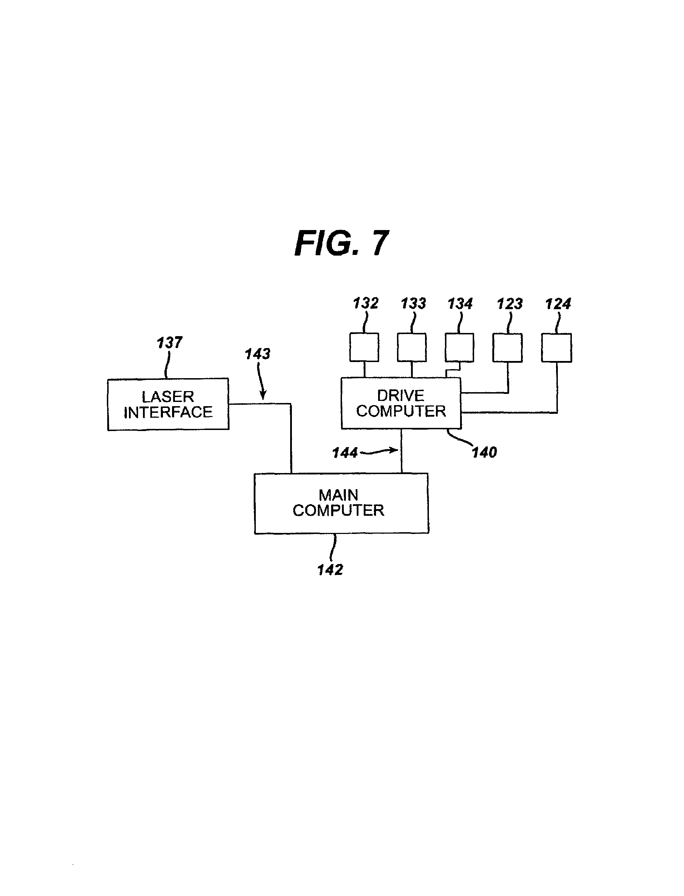

Figure 7 is a schematic illustration of the control system of the laser sculpting apparatus of Figure 6. During operation of the laser sculpting apparatus, control variables for focal position, rotational speed, and traverse speed are sent from a main computer 142 through connection 144 to a drive computer 140. The drive computer 140 controls focus position through focusing stage drive 132. Drive computer 140 controls the rotational speed of the workpiece 102 through rotational drive 123 and rotational pulse generator 124. Drive computer 140 controls the traverse speed of the carriage 126 through carriage drive 133 and carriage pulse generator 134. Drive computer 140 also reports drive status and possible errors to the main computer 142. This system provides positive position control and in effect divides the surface of the workpiece 102 into small areas called pixels, where each pixel consists of a fixed number of pulses of the rotational drive and a fixed number of pulses of the traverse drive. The main computer 142 also controls laser 137 through connection 143.

A laser sculpted three dimensional topographical support member may be made by several methods. One method of producing such a support member is by a combination of laser drilling and laser milling of the surface of a workpiece.

Methods of laser drilling a workpiece include percussion drilling. fire-on-the-fly drilling, and raster scan drilling.

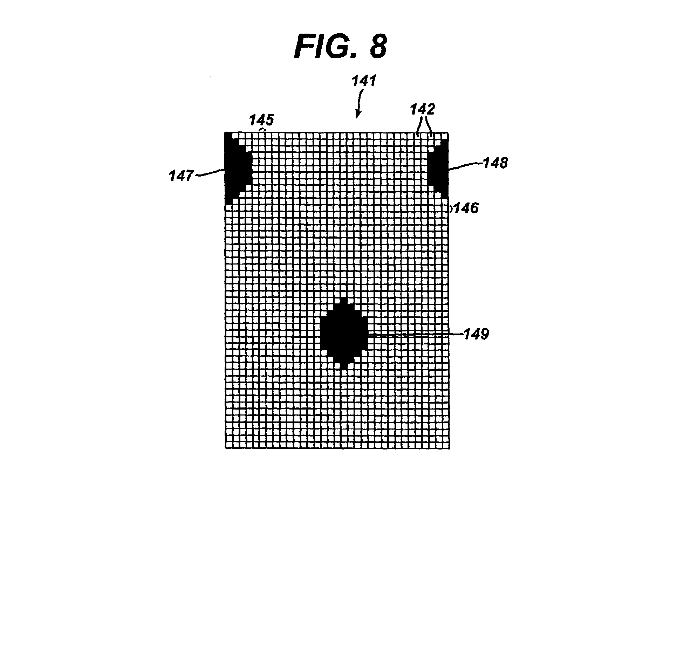

A preferred method is raster scan drilling. In this approach, the pattern is reduced to a rectangular repeat element 141 as depicted in FIG. 8. This repeat element contains all of the information required to produce the desired pattern. When used like a tile and placed both end-to-end and side-by-side, the larger desired pattern is the result.

This repeat element is further divided into a grid of smaller rectangular units or "pixels" 142. Though typically square, for some purposes, it may be more convenient to employ pixels of unequal proportions. The pixels themselves are dimensionless and the actual dimensions of the image are set during processing, that is, the width 145 of a pixel and the length 146 of a pixel are only set during the actual drilling operation. During drilling, the length of a pixel is set to a dimension that corresponds to a selected number of pulses from the carriage pulse generator 134. Similarly, the width of a pixel is set to a dimension that corresponds to the number of pulses from the rotational pulse generator 124. Thus, for ease of explanation, the pixels are shown to be square in Figure 8; however, it is not required that pixels be square, but only that they be rectangular.

Each column of pixels represents one pass of the workpiece past the focal position of the laser. This column is repeated as many times as is required to reach completely around workpiece 102. Each white pixel represents an off instruction to the laser, that is the laser is emitting no power, and each black pixel represents an on instruction to the laser, that is the laser is emitting a beam. This results in a simple binary file of 1's and 0's where a 1, or white, is an instruction for the laser to shut off and a 0, or black, is an instruction for the laser to turn on. Thus, in Figure 8, areas 147, 148 and 149 correspond to instructions for the laser to emit full power and will result in holes in the workpiece 102.

Referring back to Figure 7, the contents of an engraving file are sent in a binary form, where 1 is off and 0 is on, by the main computer 142 to the laser 137 via connection 143. By varying the time between each instruction, the duration of the instruction is adjusted to conform to the size of the pixel. After each column of the file is completed, that column is again processed, or repeated, until the entire circumference is completed. While the instructions of a column are being carried out, the traverse drive is moved slightly. The speed of traverse is set so that upon completion of a circumferential engraving, the traverse drive has moved the focusing lens the width of a column of pixels and the next column of pixels is processed. This continues until the end of the file is reached and the file is again repeated in the axial dimension until the total desired width is reached.

In this approach, each pass produces a number of narrow cuts in the material, rather than a large hole. Because these cuts are precisely registered to line up side-by-side and overlap somewhat, the cumulative effect is a hole.

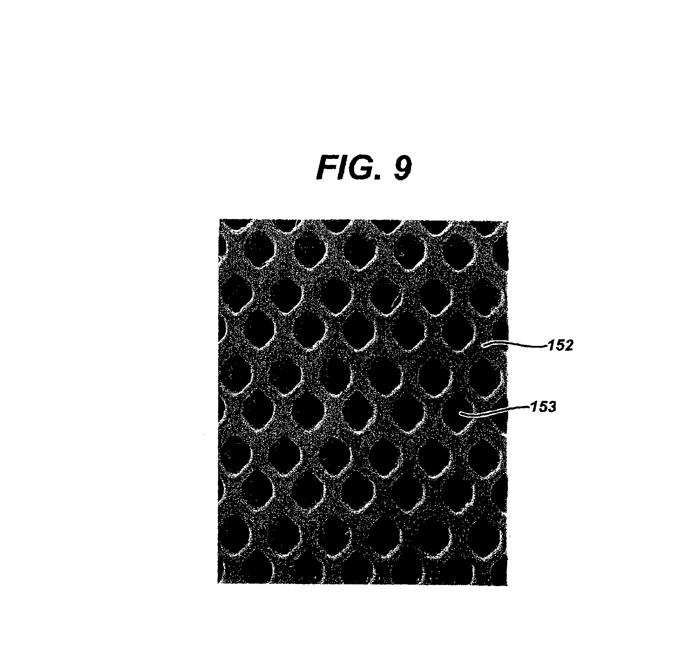

Figure 9 is a photomicrograph of a portion of a support member that has initially been raster scan drilled utilizing the file of Figure 8. The surface of the support member is a smooth planar surface 152 with a series of nested hexagonal holes 153.

A highly preferred method for making laser sculpted three dimensional topographical support members of this invention is through laser modulation. Laser modulation is carried out by gradually varying the laser power on a pixel by pixel basis. In laser modulation, the simple on or off instructions of raster scan drilling are replaced by instructions that adjust on a gradual scale the laser power for each individual pixel of the laser modulation file. In this manner a three dimensional structure can be imparted to the workpiece in a single pass over the workpiece.

Laser modulation has several advantages over other methods of producing a three-dimensional topographical support member. Laser modulation produces a one-piece, seamless, support member without the pattern mismatches caused by the presence of a seam. With laser modulation, the support member is completed in a single operation instead of multiple operations, thus increasing efficiency and decreasing cost. Laser modulation eliminates problems with the registration of patterns, which can be a problem in a multi-step sequential operation. Laser modulation also allows for the creation of topographical features with complex geometries over a substantial distance. By varying the instructions to the laser, the depth and shape of a feature can be precisely controlled and features that continuously vary in cross section can be formed. The regular positions of the apertures and macrofeatures relative to one another can be maintained.

Referring again to Figure 7, during laser modulation the main computer 142 may send instructions to the laser 137 in other than a simple "on" or "off" format. For example, the simple binary file may be replaced with an 8 bit (byte) format, which allows for a variation in power emitted by the laser of 256 possible levels. Utilizing a byte format, the instruction "11111111" instructs the laser to turn off, "00000000" instructs the laser to emit full power, and an instruction such as "10000000" instructs the laser to emit one-half of the total available laser power.

A laser modulation file can be created in many ways. One such method is to construct the file graphically using a gray scale of a 256 color level computer image. In such a gray scale image, black can represent full power and white can represent no power with the varying levels of gray in between representing intermediate power levels. A number of computer graphics programs can be used to visualize or create such a laser-sculpting file. Utilizing such a file, the power emitted by the laser is modulated on a pixel by pixel basis and can therefore directly sculpt a three dimensional topographical support member. While an 8-bit byte format is described here, other levels, such as 4 bit, 16 bit, 24 bit or other formats can be substituted.

A suitable laser for use in a laser modulation system for laser sculpting is a fast flow CO₂ laser with a power output of 2500 watts, although a laser of lower power output could be used. Of primary concern is that the laser must be able to switch power levels as quickly as possible. A preferred switching rate is at least 10 kHz and even more preferred is a rate of 20 kHz. The high power-switching rate is needed to be able to process as many pixels per second as possible.

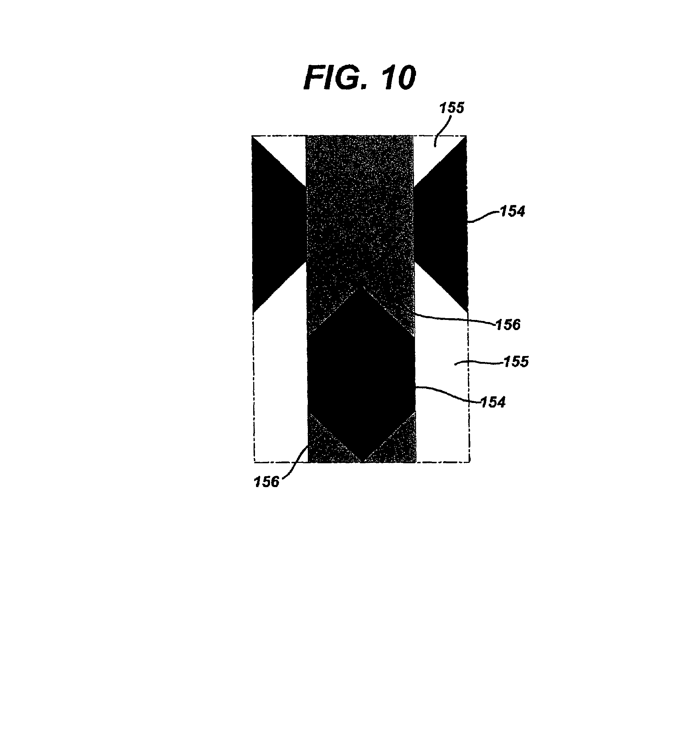

Figure 10 shows a graphical representation of a laser modulation file to produce a support member using laser modulation. The support member made with the file of Figure 10 is used to make the three-dimensional apertured film shown in Figure 2. In Figure 10, the black areas 154 indicate pixels where the laser is instructed to emit full power, thereby creating a hole in the support member, which corresponds to apertures 12 im the three-dimensional apertured film 20 illustrated in Figure 2. Likewise, white areas 155 in Figure 10 indicate pixels where the laser receives instructions to turn off, thereby leaving the surface of the support member intact. These intact areas of the support member correspond to the macrofeatures 14 of the three-dimensional apertured film 20 of Figure 2. The gray area 156 in Figure 10 indicates pixels where the laser is instructed to emit partial power and produce a lower region on the support member. This lower region on the support member corresponds to lower region 16 on the three-dimensional apertured film 20 of Figure 2.

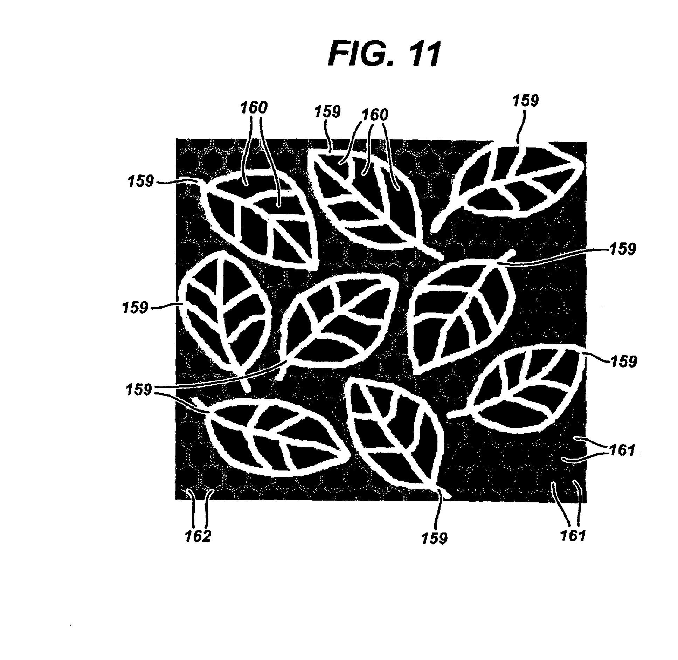

Figure 11 shows a graphical representation of a laser modulation file to produce a support member using laser modulation. As in the laser-drilling file of Figure 8, each pixel represents a position on the surface of the workpiece. Each row of pixels represents a position in the axial direction of the workpiece to be sculpted. Each column of pixels represents a position in the circumferential position of the workpiece. Unlike the file of Figure 8 however, each of the laser instructions represented by the pixels is no longer a binary instruction, but has been replaced by 8 bit or gray scale instructions. That is, each pixel has an 8-bit value, which translates to a specific power level.

Figure 11 is a graphical representation of a laser modulation file to produce a support member using laser modulation. The file shows a series of nine leaf-like structures 159, which are shown in white. The leaves are a series of white pixels and are instructions for the laser to be off and emit no power. Leaves of these shapes, therefore, would form the uppermost surface of the support member after the pattern has been sculpted into it. Each leaf structure contains a series of six holes 160, which are defined by the stem-like structures of the leaves and extend through the thickness of the workpiece. The holes 160 consist of an area of black pixels, which are instructions for the laser to emit full power and thus drill through the workpiece. The leaves are discrete macrofeatures, i.e., by themselves they do not form a flat planar structure, as no leaf interconnects with any other leaf. The background pattern of this structure consists of a close-packed staggered pattern of hexagonal black areas 161, which are also instructs for the laser to emit full power and drill a hole through the workpiece. The field 162, which defines holes 161, is at a laser power level that is neither fully on nor fully off. This produces a second planar area, which is below the uppermost surface of the workpiece as defined by the off instructions of the white areas of the leaves.

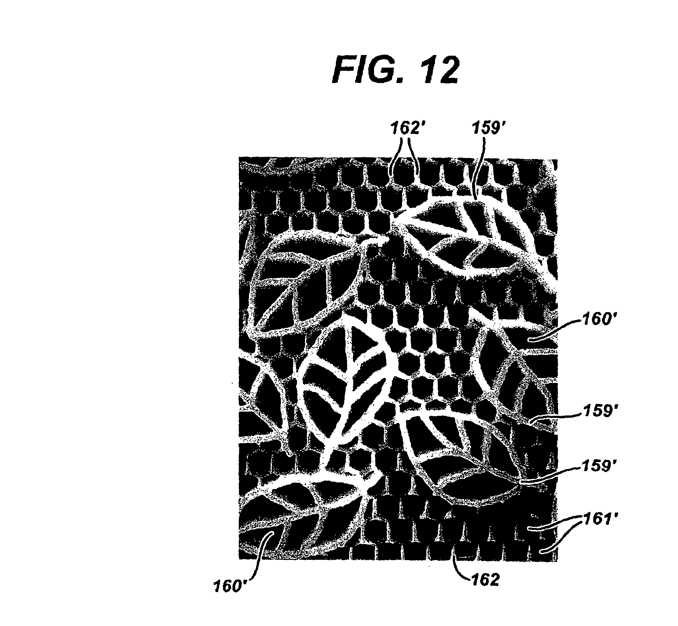

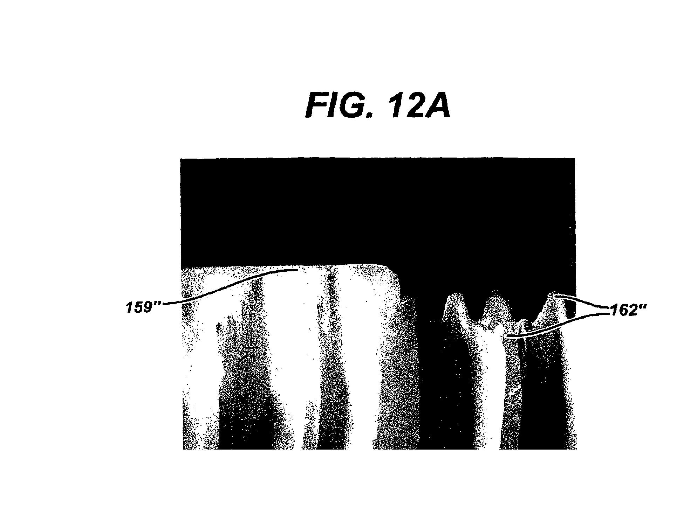

Figure 12 is a photomicrograph of a laser sculpted three dimensional topographical support member produced by laser modulation utilizing the laser modulation file depicted in Figure 11. Figure 12A is a cross-sectional view of the support member of Figure 12. Regions 159' of Figure 12 and 159" of Figure 12A correspond to the leaf 159 of Figure 11. The white pixel instructions of areas 159 of Figure 11 have resulted in the laser emitting no power during the processing of those pixels. The top surface of the leaves 159' and 159" correspond to the original surface of the workpiece. Holes 160' in Figure 12 correspond to the black pixel areas 160 of Figure 11, and in processing these pixels the laser emits full power. thus cutting holes completely through the workpiece. The background film 162' of Figure 12 and 162" of Figure 12A correspond to the pixel area 162 of Figure 11. Region 162' results from processing the pixels of Figure 11 with the laser emitting partial power. This produces an area in the support member that is lower than the original surface of the workpiece and that is thus lower than the top surface of the leaves. Accordingly, the individual leaves are discrete macrofeatures, unconnected to each other.

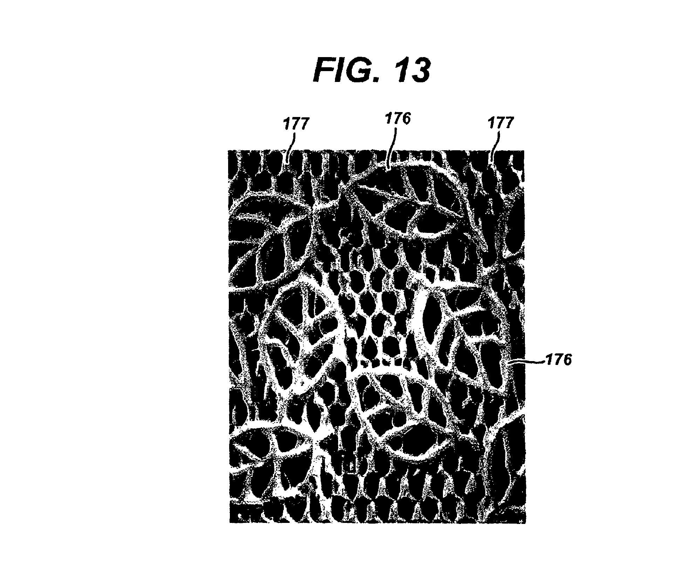

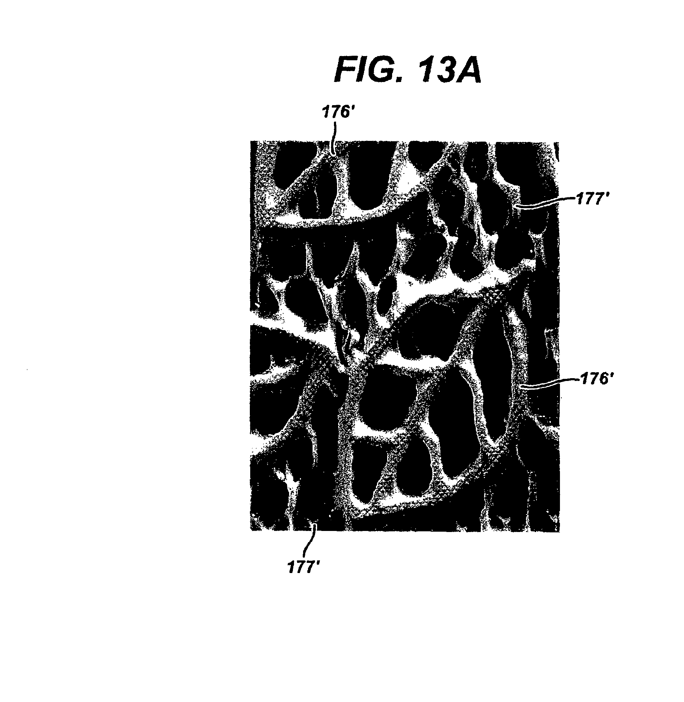

Figures 13 and 13A are photomicrographs of a three-dimensional apertured film chat has been produced on the support member of Figures 12 and 12A. The apertured film has raised apertured leaf-shaped macrofeatures 176 and 176', which correspond to the leaves 159' and 159" of the support member of Figures 12 and 12A. Each of the leaves is discrete and disconnected from all the other leaves. The plane defined by the uppermost surfaces of all the leaf shaped regions 176 and 176' is the uppermost surface of a plurality of disconnected macrofeatures. The background apertured regions 177 and 177' define a region that is at a lower depth in the film than the leaf shaped regions. This gives the visual impression that the leaves are embossed into the film.

The laser sculpted support members of Figures 9, 12, and 12A have simple geometries. That is, successive cross-sections, taken parallel to the uppermost surface of the support member, are essentially the same for a significant depth through the thickness of the support member. For example, referring to Figure 9, successive cross-sections of this support member taken parallel to the surface of the support member are essentially the same for the thickness of the support member. Similarly, cross-sections of the support member of Figures 12 and 12A are essentially the same for the depth of the leaves and are essentially the same from the base of the leaves through the thickness of the support member.

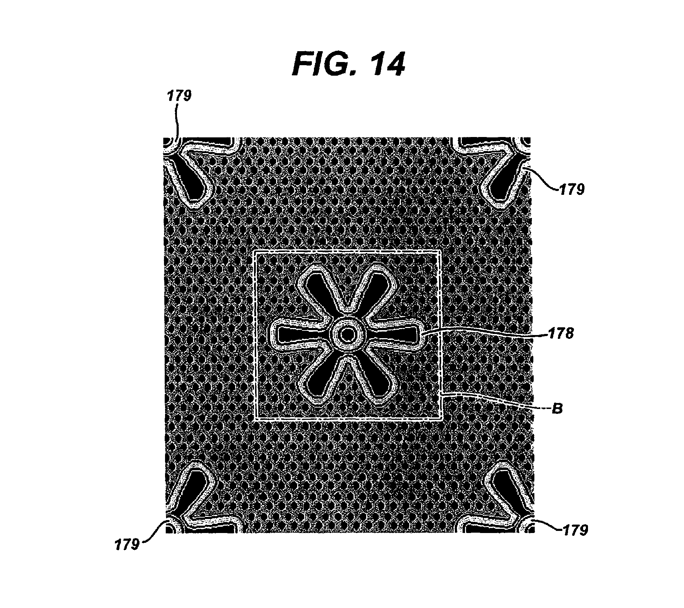

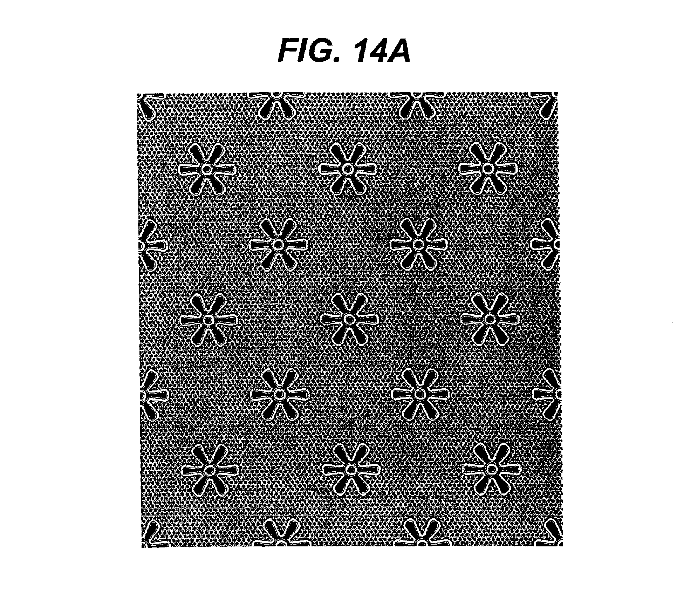

Figure 14 is a graphical representation of another laser modulation file to produce a laser sculpted support member using laser modulation. The file contains a central floral element 178 and four elements 179, each of which constitutes a quarter of a floral element 178, which combine when the file is repeated during laser sculpting. Figure 14A is a 3 repeat by 3 repeat graphical representation of the resulting pattern when the file of Figure 14 is repeated.

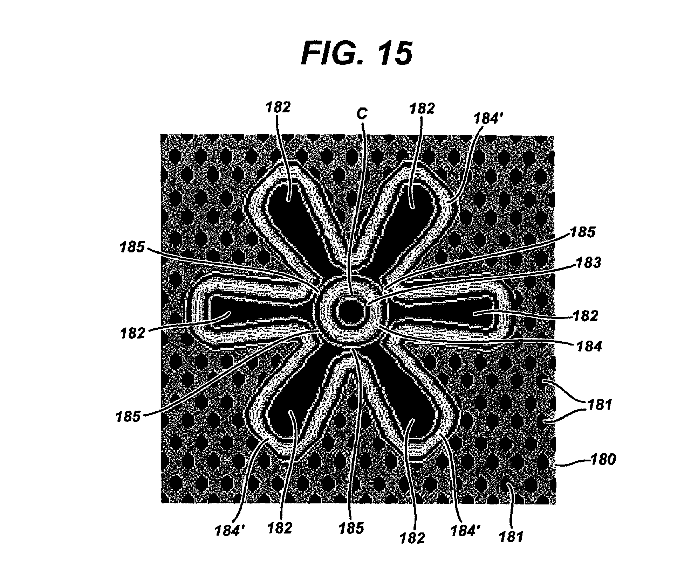

Figure 15 is a magnified view of the area B of Figure 14. The gray area represents a region of pixels instructing the laser to emit partial power. This produces a planar area below the surface of the workpiece. Contained in gray region 180 is a series of black areas 181 which are pixels instructing the laser to emit full power and drill a series of hexagonal shaped holes through the thickness of the workpiece. Central to Figure 15 is the floral element corresponding to the floral element 178 of Figure 14. The floral element consists of a center region 183 and six petal shaped regions 182 which again represent instructions for the laser to emit full power and drill a hole through the thickness of the workpiece. Defining the outside edge of the center region 183 is region 184. Defining the outside edge of the petal regions 182 is region 184'. Regions 184 and 184' represent a series of instructions for the laser to modulate the emitted power. The central black region 183 and its outside edge region 184 are joined to the region 184' by region 185 which represents instructions for the laser to emit the same power level as the background area 180.

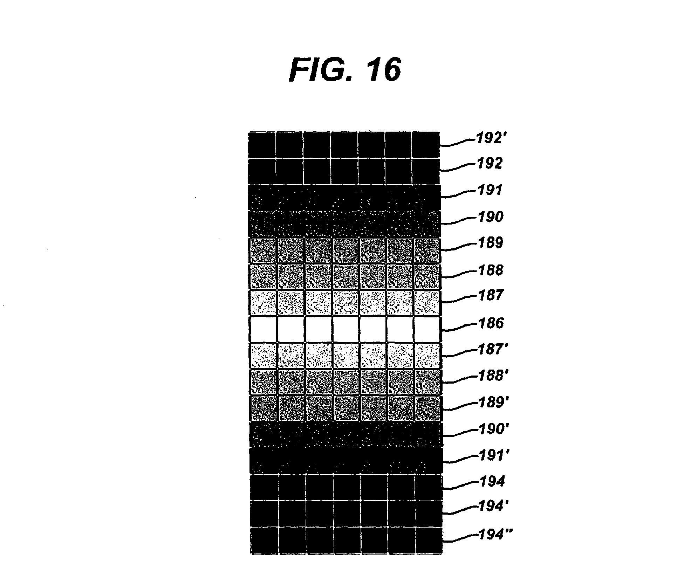

Figure 16 is an enlarged graphical representation of portion C of region 184 of Figure 15 which forms the outline of the center region 183 of Figure 15. The portion C contains a single row of white pixels 186 which instruct the laser to turn off. This defines part of the uppermost surface of the support member that remains after processing. The rows of pixels 187 and 187' instruct the laser to emit partial power. The rows 188, 189, 190, and 191 and the rows 188', 189' 190', and 191' instruct the laser to emit progressively increased levels of power. Rows 192 and 192' instruct the laser to emit the power level also represented by region 185 of Figure 15. Rows 194, 194', and 194" instruct the laser to emit full power and form part of region 183 of Figure 15.

As each column of Figure 16 is processed the laser emits the partial power represented by rows 192 and 192'. Rows 191, 190, 189, 188, and 187 instruct the laser to progressively decrease the power emitted, until row 186 is processed and the laser is instructed to not emit power. The rows 187', 188', 189', 190', and 191' then instruct the laser to again progressively increase the power emitted. Rows 194, 194', and 194" instruct the laser to again emit full power to begin drilling through the workpiece. This results in the creation of a disconnected macrofeature, which slopes from the background plane to the surface of the workpiece and then slopes back to the hole area, thus producing a radiused shape.

Depending on the size of the pixels as defined during processing, and the variation in emitted laser power for each row, the size and shape of the resulting laser sculpted feature can be changed. For example, if the variation in power level for each row of pixels is small, then a relatively shallow rounded shape is produced; conversely, if the variation in power level for each row of pixels is greater, then a deep, steep shape with a more triangular cross-section is produced. Changes in pixel size also affect the geometry of the features produced. If the pixel size is kept smaller than the actual diameter of the focused laser beam emitted, then smooth blended shapes will be produced.

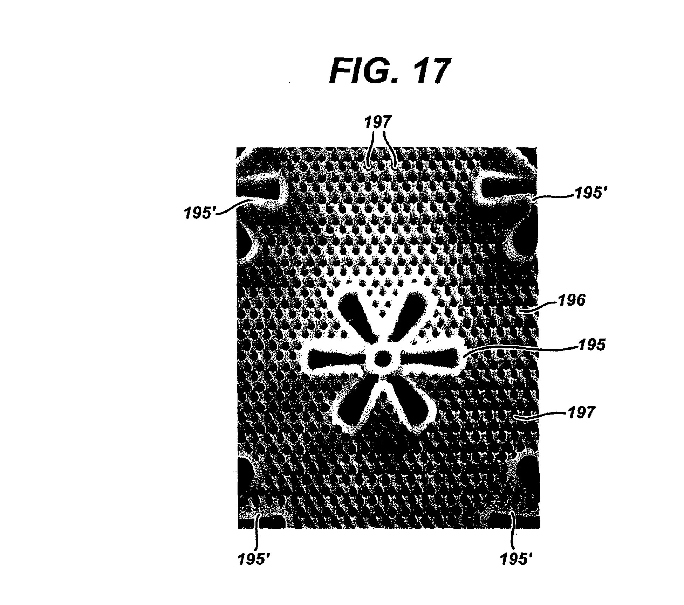

Figure 17 is a photomicrograph of the laser sculpted support member resulting from the processing of the file of Figure 14 by laser modulation. The photomicrograph shows a raised floral element 195, which corresponds to the floral element 178 of Figure 14 and the floral element of Figure 15. The photomicrograph also shows portions of additional floral elements 195'. Raised floral element 195 originates in the planar region 196, which contains holes 197. Floral elements 195 and 195' are disconnected from one another and thus do not form a continuous planar region.

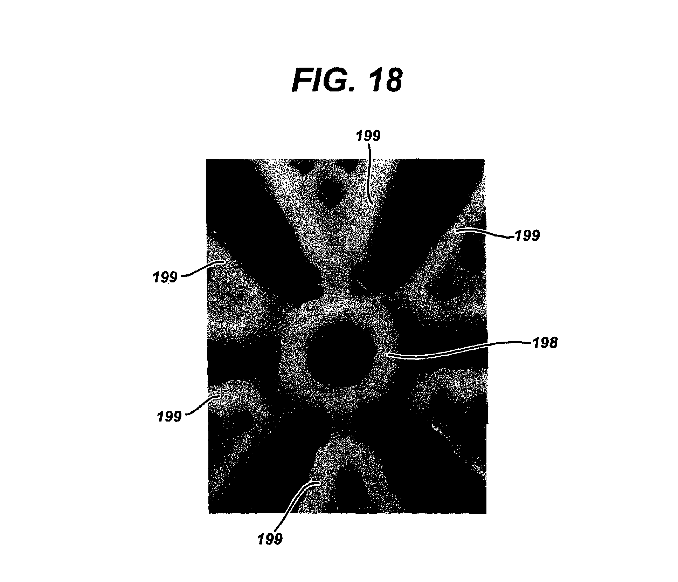

Figure 18 is an enlarged photomicrograph of a portion of the floral element 195 of Figure 17. The center circular element 198 is the area produced by the laser modulation instructions contained in region 184 of Figure 15. The elements 199 are parts of the petal elements of the floral element 195 of Figure 17. These petal elements are produced by pixel instructions depicted in region 184' of Figure 15. These elements demonstrate an example of a type of complex geometry that can be created by laser modulation. The central circular element has a semicircular cross section. That is, any one of a series of cross-sectional planes taken parallel to the original surface of the workpiece, i.e., through the depth will differ from any other of such cross-sectional planes.

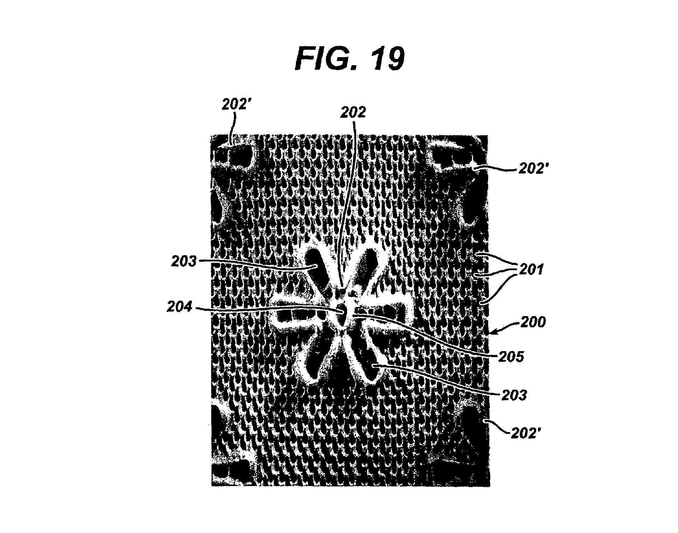

Figure 19 is a photomicrograph of the upper surface of a film produced on the support member of Figure 17. The film has an apertured planar area 200, containing holes 201 that corresponds to planar region 196 of Figure 17. Extending above the planar area are floral areas 202 and 202', which correspond to floral elements 195 and 195', respectively, of Figure 17. The floral areas 202 and 202' give the resulting apertured film an embossed appearance in a single operation. In addition, the floral areas define additional larger holes 203 and 204 to improve fluid transmission properties.

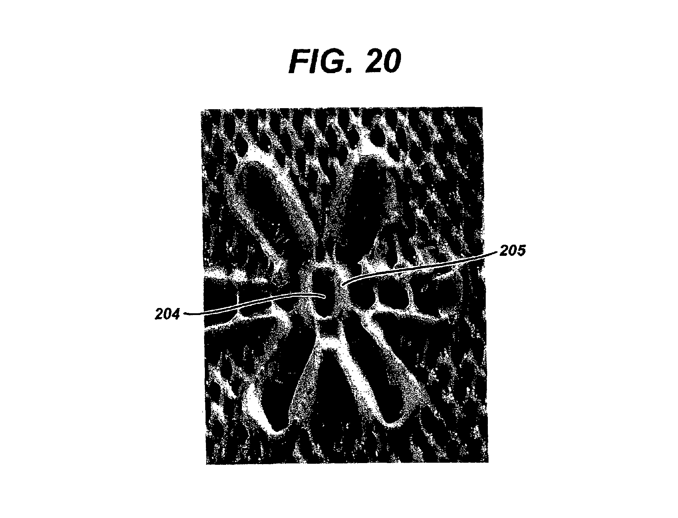

Figure 20 is an enlargement of the floral area 202 of Figure 19. The floral area comprises hole 204 and the surrounding circular element 205. Element 205 of Figures 19 and 20 has a complex geometry in that it has a semicircular cross-section. Again, successive cross-sections taken parallel to the surface of the film taken through its depth are different.

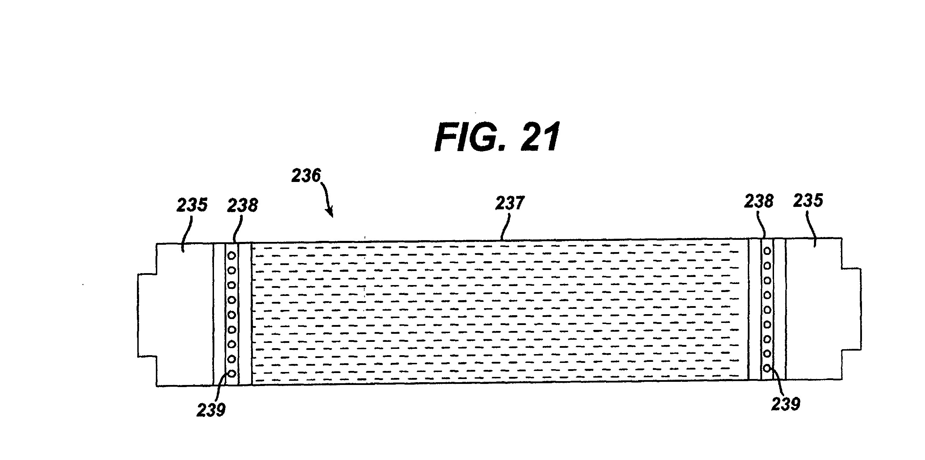

Upon completion of the laser sculpting of the workpiece, it can be assembled into the structure shown in Figure 21 for use as a support member. Two end bells 235 are fitted to the interior of the workpiece 236 with laser sculpted area 237. These end bells can be shrink-fit, press-fit, attached by mechanical means such as straps 238 and screws 239 as shown; or by other mechanical means. The end bells provide a method to keep the workpiece circular, to drive the finished assembly, and to fix the completed structure in the aperturing apparatus.

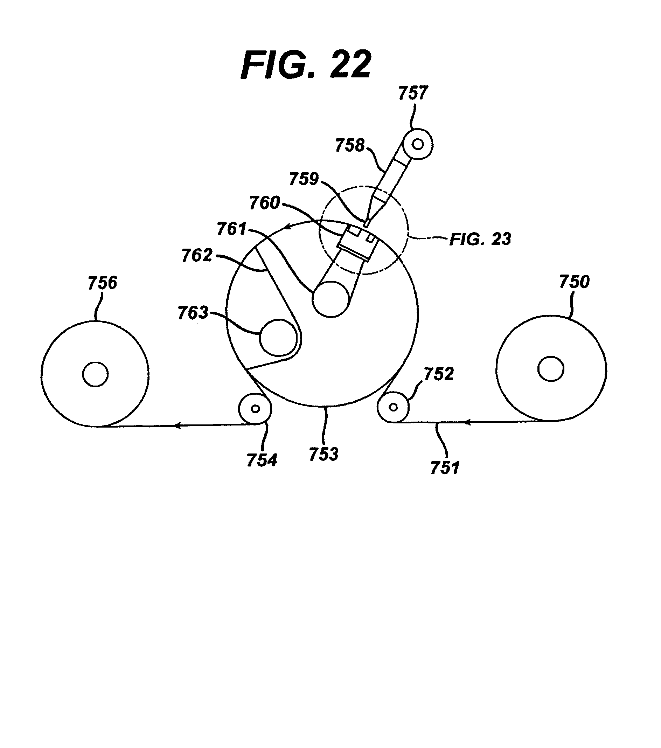

A preferred apparatus for producing apertured films in accordance with the present invention is schematically depicted in Figure 22. As shown here, the support member is a rotatable drum 753. In this particular apparatus, the drum rotates in a counterclockwise direction. Positioned outside drum 753 is a hot air nozzle 759 positioned to provide a curtain of hot air to impinge directly on the film supported by the laser sculpted support member. Means is provided to retract hot air nozzle 759 to avoid excessive heating of the film when it is stopped or moving at slow speed. Blower 757 and heater 758 cooperate to supply hot air to nozzle 759. Positioned inside the drum 753, directly opposite the nozzle 759, is vacuum head 760. Vacuum head 760 is radially adjustable and positioned so as to contact the interior surface of drum 753. A vacuum source 761 is provided to continuously exhaust vacuum head 760.

Cooling zone 762 is provided in the interior of and contacting the inner surface of drum 753. Cooling zone 762 is provided with cooling vacuum source 763. In cooling zone 762, cooling vacuum source 763 draws ambient air through the apertures made in the film to set the pattern created in the aperturing zone. Vacuum source 763 also provide means of holding the film in place in cooling zone 762 in drum 753, and provides means to isolate the film from the effects of tension produced by winding up the film after its aperturing.

Placed on top of laser sculpted support member 753 is a thin, continuous, uninterrupted film 751 of thermoplastic polymeric material.

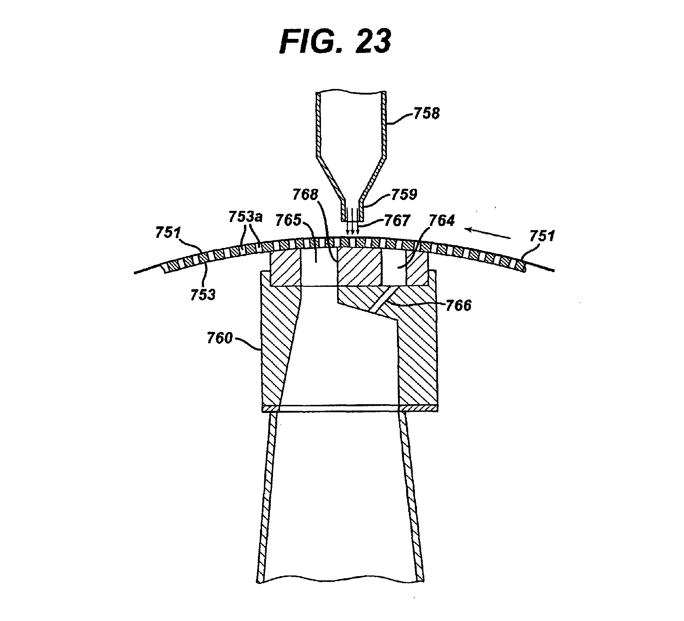

An enlargement of the circled area of Figure 22 is shown in Figure 23. As shown in this embodiment, vacuum head 760 has two vacuum slots 764 and 765 extending across the width of the film. However, for some purposes, it may be preferred to use separate vacuum sources for each vacuum slot. As shown in Figure 23, vacuum slot 764 provides a hold down zone for the starting film as it approaches air knife 758. Vacuum slot 764 is connected to a source of vacuum by a passageway 766. This anchors the incoming film 751 securely to drum 753 and provides isolation from the effects of tension in the incoming film induced by the unwinding of the film. It also flattens film 751 on the outer surface of drum 753. The second vacuum slot 765 defines the vacuum aperturing zone. Immediately between slots 764 and 765 is intermediate support bar 768. Vacuum head 760 is positioned such that the impingement point of hot air curtain 767 is directly above intermediate support bar 768. The hot air is provided at a sufficient temperature, a sufficient angle of incidence to the film, and at a sufficient distance from the film to cause the film to become softened and deformable by a force applied thereto. The geometry of the apparatus ensures that the film 751, when softened by hot air curtain 767, is isolated from tension effects by holddown slot 764 and cooling zone 762 (Figure 22). Vacuum aperturing zone 765 is immediately adjacent hot air curtain 767, which minimizes the time that the film is hot and prevents excessive heat transfer to support member 753.

Referring to Figures 22 and 23, a thin flexible film 751 is fed from a supply roll 750 over idler roll 752. Roll 752 may be attached to a load cell or other mechanism to control the feed tension of the incoming film 751. The film 751 is then placed in intimate contact with the support member 753. The film and support member then pass to vacuum zone 764. In vacuum zone 764 the differential pressure further forces the film into intimate contact with support member 753. The vacuum pressure then isolates the film from the supply tension. The film and support member combination then passes under hot air curtain 767. The hot air curtain heats the film and support member combination, thus softening the film.

The heat-softened film and the support member combination then pass into vacuum zone 765 where the heated film is deformed by the differential pressure and assumes the topography of the support member. The heated film areas that are located over open areas in the support member are further deformed into the open areas of the support member. If the heat and deformation force are sufficient, the film over the open areas of the support member is ruptured to create apertures.

The still-hot apertured film and support member combination then passes to cooling zone 762. In the cooling zone a sufficient quantity of ambient air is pulled through the now-apertured film to cool both the film and the support member.

The cooled film is then removed from the support member around idler roll 754. Idler roll 754 may be attached to a load cell or other mechanism to control winding tension. The apertured film then passes to finish roll 756, where it is wound up.

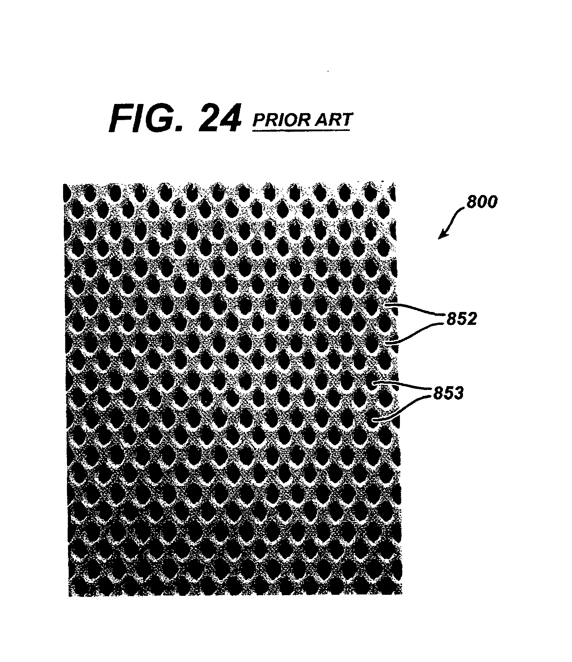

Figure 24 is a photomicrograph of an apertured film 800 of the prior art that was produced on a support member that has been raster scan drilled utilizing the file of Figure 9. The surface of this apertured film is a planar surface 852 with a series of nested hexagonal holes 853.

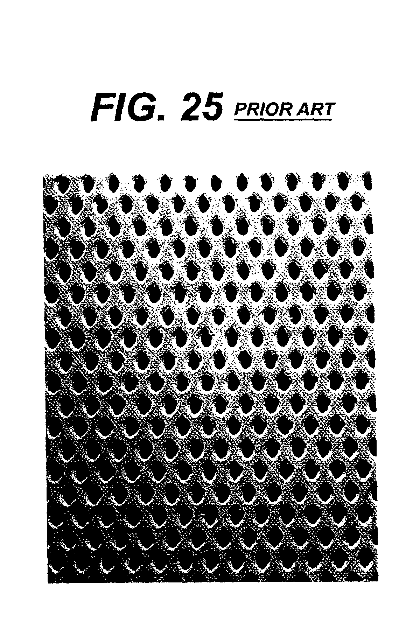

Figure 25 is a photomicrograph of another apertured film of the prior art that was produced on another support member that was produced by raster scan drilling. The surface of this apertured film is also characterized by a planar surface and a series of nested hexagonal holes that are larger than those shown in Figure 24.

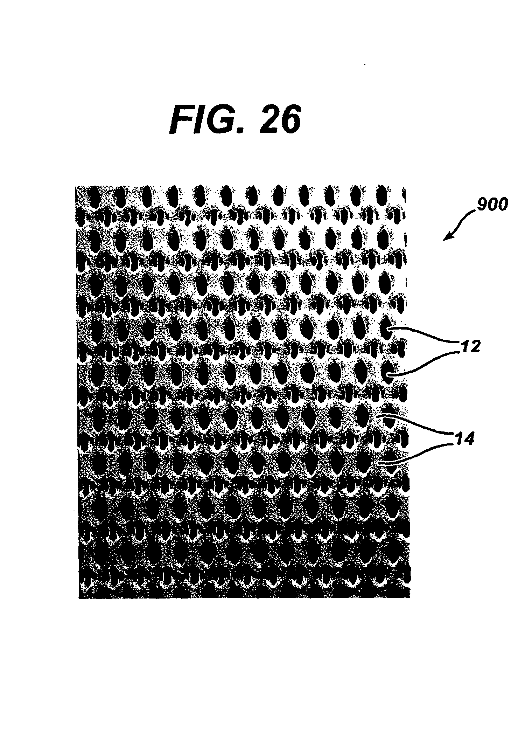

Figure 26 is a photomicrograph of a further embodiment of a three-dimensional apertured film of the present invention with an arrangement of apertures and macrofeatures. The film 900 of Figure 26 has apertures 12 arranged with macrofeatures 14. All of the apertures 12 and macrofeatures 14 are arranged together so that their relative positions to one another are regular.

While the method of forming an apertured film has been described using a hot air curtain as the mechanism to heat the film, any suitable method such as infrared heating, heated rolls, or the like may be employed to produce an apertured film using the laser sculpted three-dimensional topographical support member of this invention.

In another method for producing an apertured film the incoming film supply system can be replaced with a suitable extrusion system. In this case the extrusion system provides a film extrudate; which, depending on the extrudate temperature, can either be cooled to a suitable temperature by various means such as cold air blast or chilled roll prior to contacting the three dimensional topographical support or be brought in direct contact with the three dimensional topographical support. The film extrudate and forming surface are then subjected to the same vacuum forming forces as described above without the need to heat the film to soften the film to make it deformable.

### Examples

The present three-dimensional apertured film with a plurality of discrete macrofeatures has favorable fluid handling properties. In particular, disposable absorbent products with the film of the present invention as a component layer have a low Fluid Penetration Time. Additionally, disposable absorbent products comprising the film exhibit a Repeat Insult Time that increases less than about 40% over six insults.

Examples of the present invention and samples of apertured film of the prior art were used as a transfer layer in test assemblies made for testing using the Fluid Penetration Rate Test and the Repeat Insult Test. The test fluid used for the Liquid Penetration Test and the Repeat Insult Test was a synthetic menstrual fluid having a viscosity of 30 centipoise at 1 radian per second.

Test assemblies were made using cover layer, absorbent core and barrier from the commercially available sanitary napkin, Stayfree Ultra Thin Long with Wings, distributed by Personal Products Company Division of McMeil-PPC, Inc. Skillman, NJ. The cover layer is a thermally bonded polypropylene fabric; the absorbent core is a material containing superabsorbent polymer and the barrier is a pigmented polyethylene film. The cover layer and transfer layers were each carefully peeled away from the product exposing the absorbent core which remained adhesively attached to the barrier film. Next, a piece of transfer layer material to be tested was cut to a size approximately 200 mm long by at least the width of the absorbent core and a pressure sensitive hot melt adhesive such as HL-1471xzp commercially available from HB Fuller Corporation, St. Paul, MN 55110, was applied to the side of the transfer layer material oriented adjacent to the exposed surface of the absorbent core. Adhesive was applied to the material to be tested by transfer from release paper which was coated with approximately 1.55 gram per square meter. The transfer layer material to be tested was oriented with adhesive side toward the absorbent core and placed on top of the absorbent core. To complete the test assembly, the cover layer was placed over the transfer layer material to be tested.

Table 1 describes commercial products tested and the absorbent test assemblies made using examples of the present invention and examples representing prior art.

**Table 1**

| **Test Assemblies** | | | | |
|---|---|---|---|---|
| **Assembly** | **Cover Layer** | **Transfer Layer** | **Absorbent** | **Barrier** |
| **Commercial Sample 1** | Stayfree Ultra Thin Long with Wing, a commercial product sold in the U.S.A by Personal Products Company, Inc. | | | |
| **Commercial Sample 2** | Always Ultra Long with Flexi-Wing, a commercial product sold in the U.S.A. by Procter & Gamble, Inc. | | | |
| **Prior Art 1** | Cover Layer¹ | Material of Fig. 24 | Absorbent Core² | Barrier³ |
| **Prior Art 2** | Cover Layer1 | Material of Fig. 25 | Absorbent Core² | Barrier³ |
| **Example 1** | Cover Layer1 | Material of Fig. 26 | Absorbent Core² | Barrier³ |
| **Example 2** | Cover Layer1 | Material of Fig. 2 | Absorbent Core² | Barrier³ |
| **Example 3** | Cover Layer1 | Material of Fig. 1 | Absorbent Core² | Barrier³ |
| Note 1: Cover Layer is the Cover Layer of Commercial Sample 1 Note 2: Absorbent Core is the absorbent core of Commercial Sample 1 Note 3: Barrier is the Barrier Layer of Commercial Sample 1 | | | | |

Fluid Penetration Time and Repeat Insult Time measured according to the following test methods, respectively. Testing was performed in a location conditioned to 21 degrees centigrade and 65% relative humidity. Prior to performing the tests, the commercial samples and test assemblies were conditioned at for at least 8 hours.

Fluid Penetration Time (FPT) is measured by placing a sample to be tested under a Fluid Penetration Test orifice plate. The orifice plate consists of a 7.6 cm X 25.4 cm plate of 1.3 cm thick polycarbonate with an elliptical orifice in its center. The elliptical orifice measures 3.8 cm along its major axis and 1.9 cm along its minor axis. The orifice plate is centered on the sample to be tested. A graduated 10 cc syringe containing 7 ml of test fluid is held over the orifice plate such that the exit of the syringe is approximately 3 inches above the orifice. The syringe is held horizontally, parallel to the surface of the test plate, the fluid is then expelled from the syringe at a rate that allows the fluid to flow in a stream vertical to the test plate into the orifice and a stop watch is started when the fluid first touches the sample to be tested. The stop watch is stopped when the surface of the sample first becomes visible within the orifice. The elapsed time on the stop watch is the Fluid Penetration Time. The average Fluid Penetration Time(FPT) is calculated from the results of testing five samples.

**TABLE 2**

| **AVERAGE FLUID PENETRATION TIME** | |
|---|---|
| **SAMPLE** | **FPT, seconds** |
| | |
| PRIOR ART 1 | 82.6 |
| EXAMPLE 1 | 59.3 |
| | |
| PRIOR ART 2 | 62.3 |
| EXAMPLE 2 | 42.2 |

The Repeat Insult Time is measured by placing a sample to be tested on a Resilient Cushion, covering the sample with a Repeat Insult Orifice Plate, then applying test fluid according to the schedule described.

The Resilient Cushion is made as follows: a nonwoven fabric of low density (0.03-0.0 g/cm3, measured at 0.24 kPa or 0.035 psi) is used as a resilient material. The nonwoven fabric is cut into rectangular sheets (32x14cm) which are placed one on top of another until a stack with a free height of about 5 cm. is reached. The nonwoven fabric stack is then wrapped with one layer of 0.01 mm thick polyurethane elastomeric film such as TUFTANE film (manufactured by Lord Corp., UK) which is sealed on the back with double-face clear tape.

The Repeat Insult orifice plate consists of a 7.6 cm X 25.4 cm plate of 1.3 cm thick polycarbonate with a circular orifice in its center. The diameter of the circular orifice is 2.0 cm. The orifice plate is centered on the sample to be tested. A graduated 10 cc syringe containing 2 ml of test fluid is held over the orifice plate such that the exit of the syringe is approximately 1 inch above the orifice. The syringe is held horizontally, parallel to the surface of the test plate, the fluid is then expelled from the syringe at a rate that allows the fluid to flow in a stream vertical to the test plate into the orifice and a stop watch is started when the test fluid first touches the sample to be tested. The stop watch is stopped when the surface of the sample first becomes visible within the orifice. The elapsed time on the stop watch is the first fluid penetration time. After an interval of 5 minutes elapsed time, a second 2 ml of test fluid is expelled from the syringe into the circular orifice of the Repeat Insult Orifice Plate and timed as previously described to obtain a second fluid penetration time. This sequence is repeated until a total of six fluid insults, each separated by 5 minutes, have been timed. The Percent Increase in Fluid Penetration Time after Six Insults is calculated as: 100 times the difference between the first and sixth insult times divided by the first insult time. The Average Percent Increase in Fluid Penetration Time is calculated from the results of testing five samples.

While several embodiments and variations of the present invention are described in detail herein, it should be apparent that the disclosure and teachings of the present invention will suggest many alternative designs to those skilled in the art.

## Claims

1. A three-dimensional apertured film comprising a first surface, a second surface, and a caliper defined by a first plane and a second plane, said film comprising a plurality of disconnected macrofeatures and a plurality of apertures, said apertures defined by sidewalls originating in the first surface and extending generally in the direction of the second surface and terminating in the second plane, wherein the first surface is coincident with the first plane at said macrofeatures and the relative positions of said apertures and macrofeatures is regular.

2. The film according to claim 1, wherein at least a portion of said sidewalls have a first portion thereof originating in said first plane.

3. The film according to claim 2, wherein at least 50% of said sidewalls have a first portion thereof originating in said first plane.

4. The film according to claim 2, wherein at least a portion of said sidewalls have a second portion originating between the first plane and the second plane.

5. The film according to claim 1 wherein the ratio of apertures to macrofeatures is at least one.

6. An absorbent article comprising a three-dimensional apertured film comprising a first surface, a second surface, and a caliper defined by a first plane and a second plane, said film comprising a plurality of disconnected macrofeatures and a plurality of apertures, said apertures defined by sidewalls originating in the first surface and extending generally in the direction of the second surface and terminating in the second plane, wherein the first surface is coincident with the first plane at said macrofeatures and the relative positions of said apertures and macrofeatures is regular.

7. The absorbent article according to claim 6 further comprising an absorbent core with a body-facing surface wherein the three-dimensional film is adjacent to the body-facing surface of the absorbent core.

8. The absorbent article according to claim 6, wherein at least a portion of said sidewalls have a first portion thereof originating in said first plane.

9. The absorbent article according to claim 6, wherein at least 50% of said sidewalls have a first portion thereof originating in said first plane.

10. The absorbent article according to claim 8, wherein at least a portion of said sidewalls have a second portion originating between the first plane and the second plane.

11. The absorbent article according to claim 6 wherein the ratio of apertures to macrofeatures is at least one.

12. An absorbent article comprising a nonwoven cover layer, an intermediate layer and an absorbent core comprising superabsorbent polymer wherein the absorbent article has a repeat insult time which increases less than 40% over six insults.

13. An absorbent article according to claim 12 wherein the repeat insult time increases less than 30% over six insults.

14. An absorbent article according to claim 12 wherein the repeat insult time increases less than 20% over six insults.

15. An absorbent article according to claim 12 wherein the repeat insult time increases less than 15% over six insults.
